Europäisches Patentamt

European Patent Office

Office européen des brevets

(19)

(11) Veröffentlichungsnummer: **0 412 192 A1**

# (12) EUROPÄISCHE PATENTANMELDUNG

(21) Anmeldenummer: 89114829.8

(22) Anmeldetag: 10.08.89

(51) Int. Cl.⁵: **C07C 45/75, C08G 2/08**

(43) Veröffentlichungstag der Anmeldung:
**13.02.91 Patentblatt 91/07**

(84) Benannte Vertragsstaaten:
**AT BE CH DE ES FR GB GR IT LI LU NL SE**

(71) Anmelder: **VEB Leuna-Werke "Walter Ulbricht"**

**O-4220 Leuna 3(DE)**

(72) Erfinder: **Bühner, Rolf-Dirk, Dipl.-Ing.**
**Klingenthaler Strasse 14**
**D-4015 Halle(DD)**
Erfinder: **Organo, Werner, Dipl.-Ing.**
**B.-Koenen-Strasse 3**
**D-4070 Halle(DD)**
Erfinder: **Pieron, Wolfgang, Chem.-Ing.**
**Thälmann-Platz 26**
**D-4220 Leuna(DD)**
Erfinder: **Müller, Heinz, Dr. Dipl.-Chem.**
**Nelkenweg 6**
**D-4220 Launa(DD)**
Erfinder: **Würker, Klaus, Dipl.-Ing.**
**A.-Einstein-Strasse 10**
**D-4220 Launa(DD)**
Erfinder: **Koinzer, Jürgen-Peter, Dr. Dipl.-Chem.**

**D-4090 Halle-Neustadt, 461/7(DD)**
Erfinder: **Wurbs, Adolf, Dr. Dipl.-Chem.**
**Prager Strasse 27**
**D-4070 Halle(DD)**

(54) Verfahren zur Herstellung von körnigem Paraformaldehyd.

(57) Die Erfindung betrifft ein Verfahren zur Herstellung von körnigem Paraformaldehyd durch Versprühen eines heißen, wäßrigen 83 bis 90 %-igen Formaldehydkonzentrats in einem Sprühturm, der mit einer Wirbelschicht kombiniert ist, wobei erfindungsgemäß die Abkühlung der versprühten Formaldehydkonzentrattröpfchen durch einen Gasstrom erfolgt, dessen Anfangstemperatur $\leq 303$ K beträgt und dessen Anfangsfeuchte unterhalb des Taupunktes liegt und der aus dem Fluidisierungsgas für die Wirbelschicht und einem oberhalb der Wirbelschicht zugegebenen Gasstrom besteht. Die gebildeten Paraformaldehydteilchen werden dann in einer Wirbelschicht mit einem temperierten Fluidisierungsgas, dessen Feuchtegehalt vor Eintritt in die Wirbelschicht maximal 50 % beträgt, mit einer mittleren Verweilzeit von mindestens 15 min fluidisiert, wobei ihre Temperatur zwischen 313 und 323 K gehalten wird.

Das Verfahren zeichnet sich durch geringen apparativen und energetischen Aufwand, einfache Durchführbarkeit und geringe apparative Ansprüche aus und führt zu einem freifließenden Paraformaldehyd mit einem Formaldehydgehalt $\geq 90$ Masse-% ohne Verklebungen bei der Prozeßführung.

## VERFAHREN ZUR HERSTELLUNG VON KÖRNIGEM PARAFORMALDEHYD

Die Erfindung betrifft bin Verfahren zur Herstellung eines freifließenden körnigen Paraformaldehyds hoher Reaktivität und Alterungsheständigkeit mit einem Formaldehydgehalt von ≥ 90 Masse-% aus einem wäßrigen Formaldehydkonzentrat.

Es ist bekannt, daß man einen freifließenden, körnigen Paraformaldehyd herstellen kann, indem ein wäßriges Formaldehydkonzentrat mit einem Gehalt von 80 bis 90 Masse-% Formaldehyd in einem kombinierten Wirbelschichtsprühtrockner versprüht und kurze Zeit in der Wirbelschicht durch entgegenströmende Warmluft von 313 bis 333 K nachgetrocknet wird (DE-A-1 208 887. Das so hergestellte Produkt hat einen Formaldehydgehalt von ca. 88 Masse-%. Dieses Verfahren wurde bereits in DE-A-1 720 472 als im technischen Maßstab nicht durchführbar bezeichnet, da die Oberfläche der primär entstehenden kleinen Paraformaldehydkügelchen nicht genügend klebfrei ist, es infolgedessen zur Agglomerierung und nach kurzer Zeit zu Verklebungen besonders des Anströmbodens des Wirbelschichttrockners kommt. Dieser Nachteil wird nach DE-A-1 720 472 dadurch behoben, daß die Temperatur des entgegenströmenden Gasen unterhalb 312K und vorzugsweise zwischen 253 und 293K gehalten wird, wo bei die entstehenden Perlen den Turm mit einer Temperatur von < 313K verlassen. Das Verfahren kann mit und ohne Wirbelschicht betrieben werden, wobei das Gas unterhalb des Anströmbodens in den Sprühtrockner eingeleitet wird. Das Produkt hat nach einer zwölfstündigen Zwischenlagerung in einem Bunker einen Formaldehydgehalt von 90 %. In der Praxis hat sich gezeigt, daß sich auch nach dieser Verfahrensweise Agglomerate bilden und es zu Verklebungen im unteren Teil des Turmes sowie zur Klumpenbildung in der ggfs. erzeugten Wirbelschicht kommt, da die in kurzer Zeit auf vorzugsweise 293 bis 303 K abgekühlten Perlen klebrig anfallen. Außerdem ist der Formaldehydgehalt bei der Entnahme der Perlen aus dem Turm nur geringfügig gegenüber dem Ausgangsprodukt erhöht.

Aus DE-A-1 768 951 ist weiterhin bekannt, daß man auch durch Versprühen einer 75 bis 93 %-igen (im Beispiel einer 79 %-igen) wäßrigen Formaldehydlösung unter Zusatz eines Amins als Katalysator und Abkühlung des Sprühprodukts auf 253 bis 333 K durch Einleiten von inerten Gasen in den Sprühturm einen freifließenden, körnigen Paraformaldehyd mit einem Formaldehydgehalt erhält, der dem des Ausgangskonzentrats entspricht. Der Formaldehydgehalt kann durch weiteres Behandeln in Heißlufttrocknern im Wirbelschichtverfahren oder in Etagentrocknern unter vermindertem Druck auf einen Formaldehydgehalt von 92 bis 99 Masse-% gebracht werden, wobei jedoch mit größeren Formaldehydverlusten zu rechnen ist. Ein weiterer Nachteil dieses Verfahrens ist der verbleibende Gehalt an Amin-Katalysatoren im Endprodukt, der auf manchen Einsatzgebieten zu unerwünschten Nebenreaktionen, z.B. zur Bildung zuckerartiger Produkte, führt.

In DE-A-1 914 269 ist ebenfalls ein Sprühverfahren zur Herstellung von gekörntem Paraformaldehyd beschrieben) das mit einem sauren bzw. basischen Katalysator arbeitet. Um ein trockenes Produkt mit einem Formaldehydgehalt ≥92 Masse-% zu erhalten, wird das zunächst in ein Wirbelbett mit einer Temperatur von 303 K versprühte Produkt anschließend in zwei nachgeschalteten Türmen bei Temperaturen von 303 bis 333 K über 2 bis 12 h weiter polykondensiert und nachgetrocknet. Zur Vermeidung einer Agglomerierung in der Wirbelschicht am Boden des Sprühturmes muß diese zum Teil aus zuvor getrockneten Körnern bestehen. Dieses Verfahren besitzt die bereits genannten Nachteile, die in größeren Formaldehydverlusten bei der Nachtrocknung sowie im Gehalt an Katalysatoren im Endprodukt bestehen. Außerdem wird es mit dem hohen verfahrenstechnischen Aufwand der Stufen Versprühen, Wirbelschichttrocknung, Polymerisationsturm und Nachtrocknung durchgeführt, wobei durch die lange Verweilzeit große Apparate erforderlich sind.

Der Erfindung liegt die Aufgabe zugrunde, die Herstellung von Paraformaldehyd aus wäßrigen Formaldehydkonzentraten mit einem Formaldehydgehalt von 83 bis 90 Masse-% unter Anwendung eines Wirbelschichtverfahrens in einer einfachen Apparatur ohne Einsatz von Katalysatoren und bei einfacher Verfahrensweise und geringem Energieaufwand so zu gestalten, daß das Produkt körnig, klebfrei und freifließend ist, eine hohe Reaktivität und einen Formaldehydgehalt von mindestens 90 Masse-% besitzt und keine Verklebungen in der Wirbelschicht und im Austragssystem auftreten.

Diese Aufgabe wird anspruchsgemäß gelöst. Die abhängigen Ansprüche betreffen vorteilhafte Ausführungsformen.

Beim erfindungsgemäßen Verfahren wird ein heißes, wäßriges Formaldehydkonzentrat mit einem Formaldehydgehalt von 83 bis 90 Masse-% ohne Katalysatorzusatz in einem Sprühturm, der mit einer Wirbelschicht kombiniert ist, versprüht, wobei erfindungsgemäß die Abkühlung der versprühten Formaldehydkonzentrattröpfchen durch einen Gasstrom erfolgt, dessen Anfangstemperatur ≤ 303 K beträgt und dessen Anfangsfeuchte unterhalb des Taupunktes liegt und der sich aus dem Fluidisierungsgas für die Wirbelschicht und einem oberhalb der Wirbelschicht zugeführten Gasstrom zusammensetzt, und die dabei gebildeten Paraformaldehydteilchen anschließend mittels eines temperierten Fluidisierungsgases, dessen relativer Feuchtegehalt vor Eintritt in die Wirbelschicht maximal 50 beträgt, mit einer mittleren Verweilzeit von mindestens 15 min in der Wirbelschicht fluidisiert werden, wobei ihre Temperatur auf 313 bis 323 K gehalten wird.

Durch diese Verfahrensweise werden einmal die versprühten Konzentrattröpfchen mittels des relativ kalten, nicht wassergesättigten Gasstromes beim Absinken im Sprühturm gekühlt, zu Prills verfestigt und oberflächlich teilweise getrocknet, und zum anderen wird durch die relativ hohe Temperatur der Prills in der Wirbelschicht und die hohe Wasseraufnahmefähigkeit des Fluidisierungsgases das Wasser schnell von der Oberfläche der Prills entfernt und die Diffusion des Wassers aus dem Inneren der Prills an deren Oberfläche gefördert. Es zeigt sich, daß trotz des hohen Wasseranfalls pro Zeiteinheit an der Oberfläche der Prills, der durch die Erhöhung des Formaldehydgehaltes der Prills auf mindestens 90 Masse-% bedingt ist, das Wirbelbett ohne Agglomeratbildung betrieben werden kann.

Es zeigt sich weiterhin, daß durch diese Verfahrensweise die Formaldehydverluste gegenüber anderen Verfahren stark reduziert werden, da die Wasserentfernung von den Prills durch den niedrigen Wassergehalt im Fluidisierungsgas bei einer relativ niedrigen Temperatur im Vergleich zu anderen bekannten Trocknungsverfahren, wie z.B. mit Heißluftwirbelbetttrockner und Etagentrockner, abläuft und außerdem das Fluidisierungsgas, welches geringe Mengen Formaldehyd aufnimmt, auch mit zur Kühlung der Konzentrattröpfchen eingesetzt wird, wodurch der Formaldehydübergang in das Kühlgas gegenüber eingesetztem Frischgas abgesenkt wird. Ein weiterer Vorteil der Verfahrensweise, der durch die Verwendung des Fluidisierungsgases als Kühlgas hervorgerufen wird, ist die geringe Gasmenge, die zu fördern und ggfs. vor der Abgabe an die Umwelt von Formaldehyd zu reinigen ist, wodurch sich kleinere Apparate und Aggregate sowie ein geringerer Energieaufwand ergeben.

Die erfindungsgemäße Verfahrensweise ermöglicht somit, auf einfache Art und Weise aus einem wäßrigen Formaldehydkonzentrat mit 83 bis 90 Masse-% Formaldehyd einen körnigen, freifließenden Paraformaldehyd mit einem Formaldehydgehalt von mindestens 90 Masse-% herzustellen. Als Fluidisierungs- und Kühlgas können Luft, Stickstoff und andere inerte Gase eingesetzt werden Die Gase können im Kreislauf geführt werden oder, was insbesondere beim Einsatz von Luft zweckmäßig sein kann, nur einmal durch den Sprühturm mit der Wirbelschicht geleitet werden.

Die Aufteilung des Gesamtgasstromes in das Fluidisierungsgas und den zweiten zugeführten Gasstrom und der relative Feuchtegehalt des Fluidisierungsgases können in Abhängigkeit vom Wassergehalt des eingesetzten wäßrigen Formaldehydkonzentrates variiert werden. Eine besonders vorteilhafte Anwendung der erfindungsgemäßen Verfahrensweise wurde bei einem relativen Feuchtegehalt des Fluidisierungsgases von < 20 % gefunden. Wesentlich für das Betreiben ist jedoch in jedem Fall, daß der Feuchtegehalt des Gesamtgasstromes nach dem Mischen unterhalb des Taupunktes liegt. Des weiteren beeinflußt die Temperatur der Prills in der Wirbelschicht die erforderliche Verweilzeit in ihr. Es wurde gefunden, daß die erfindungsgemäße Verfahrensweise besonders vorteilhaft ist, wenn eine mittlere Verweilzeit der Prills in der Wirbelschicht von 20 bis 30 min eingestellt wird.

Das erfindungsgemäße Verfahren wird im folgenden anhand von Ausführungsbeispielen näher erläutert.

**Beispiel 1**

In einem zylindrischen Sprühturm mit einem Durchmesser von 1,2 m und einer Höhe von 12 m werden kontinuierlich 70 kg/h wäßrige Formaldehydlösung mit einem Formaldehydgehalt von 83,9 Masse-% und einer Temperatur von 358 K über eine Einstoffvollkegeldüse mit einer Bohrung von 1,2 mm versprüht. Am Fuß des Turmes befindet sich ein Wirbelbett, dessen Anströmboden einen Durchmesser von 0,4 m hat. Durch den Anströmboden wird eine Luftmenge von 300 m³/h mit einer Temperatur von 327 K und einem Wassergehalt von 9 g/kg Luft eingeleitet. Oberhalb des Wirbelbettes werden 300 m³/h wassergesättigte Luft mit einer Temperatur von 286 K zugeführt. Im Wirbelbett herscht eine mittlere Temperatur von 320 K; die Temperatur des Gemisches aus Fluidisierungsluft und oberhalb des Wirbelbettes eingeleiteter Luft beträgt im unteren Viertel des Sprühturmes ca. 299 K. Aus dem Wirbelbett werden kontinuierlich 63,6 kg/h körniger, klebfreier und rieselfähiger Paraformaldehyd mit einem Formaldehydgehalt von 91,4 Masse-% und einem mittleren Korndurchmesser von 0,5 mm ausgetragen und in ein Vorratssilo geleitet. Die mittlere Verweilzeit des Paraformaldehydes im Wirbelbett beträgt 25 min. Beim Betreiben der Anlage treten keine Agglomerate und Verklebungen in der Wirbelschicht auf, und das Produkt läßt sich ohne Schwierigkeiten austragen.

**Beispiel 2**

In der in Beispiel 1 beschriebenen Apparatur werden 80 kg/h wäßrige Formaldehydlösung mit einem Formaldehydgehalt von 87,1 Masse-% bei einer Temperatur von 365 K versprüht. Durch den Anströmboden werden 300 m³/h Luft mit einer Temperatur von 322 K und einem Wassergehalt von 8,5 g/kg Luft eingeleitet. Oberhalb des Wirbelbettes werden 330 m³/h wassergesättigte Luft mit einer Temperatur von 285,5 K zugeführt. Im Wirbelbett herrscht eine mittlere Temperatur von 319 K, die Gastemperatur im unteren Viertel des Turmes liegt bei 299 K. Es werden kontinuierlich 75,5 kg/h Paraformaldehyd mit einem Formaldehydgehalt von 91,2 Masse-% ausgetragen. Die mittlere Verweilzeit im Wirbelbett beträgt 22 min. Das Produkt ist klebfrei und rieselfähig, Agglomerate und Verklebungen treten im Wirbelbett nicht auf.

**Ansprüche**

1. Verfahren zur Herstellung von körnigem Paraformaldehyd durch Versprühen eines heißen, wäßrigen Formaldehydkonzentrates mit einem Formaldehydgehalt von 83 bis 90 Masse-% in einem Sprühturm, der mit einer Wirbelschicht kombiniert ist,
**dadurch gekennzeichnet,**
daß die Abkühlung der versprühten Formaldehydkonzentrattröpfchen mit einem Gasstrom vorgenommen wird, dessen Anfangstemperatur $\leq$ 303 K beträgt und dessen Anfangsfeuchte unterhalb des Taupunktes liegt und der sich aus dem Fluidisierungsgas für die Wirbelschicht und einem oberhalb der Wirbelschicht zugeführten Gasstrom zusammensetzt, und die dabei gebildeten Paraformaldehydteilchen anschließend mittels eines temperierten Fluidisierungsgases, dessen relativer Feuchtegehalt vor Eintritt in die Wirbelschicht maximal 50 % beträgt, mit einer mittleren Verweilzeit von mindestens 15 min in der Wirbelschicht fluidisiert werden, wobei ihre Temperatur auf 313 bis 323 K gehalten wird.

2. Verfahren nach Anspruch 1, dadurch gekennzeichnet, daß ein Fluidisierungsgas verwendet wird, dessen relativer Feuchtegehalt unterhalb 20 % liegt.

3. Verfahren nach Anspruch 1 oder 2, dadurch gekennzeichnet, daß die Paraformaldehydteilchen durchschnittlich 20 bis 30 min fluidisiert werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| E | DD-A- 272 576 (VEB LEUNA WERKE) <br> * Ansprüche * <br> --- | 1-3 | C 07 C 45/75 <br> C 08 G 2/08 |
| A | US-A-4 036 891 (MOLLER) <br> * Spalte 1, Zeilen 38-40 * <br> --- | 1 | |
| D,A | DE-A-1 914 269 (CHEMCELL LTD) <br> * Anspruch 1 * <br> --- | 1 | |
| A | US-A-4 550 213 (THIGPEN) <br> * Anspruch * <br> --- | 1 | |
| D,A | DE-B-1 720 472 (DEUTSCHE GOLD UND SILBER SCHEIDEANSTALT) <br> * Ansprüche * <br> --- | 1 | |
| D,A | DE-B-1 208 887 (DEUTSCHE GOLD UND SILBER SCHEIDEANSTALT) <br> * Ansprüche * <br> ----- | 1 | |

**RECHERCHIERTE SACHGEBIETE (Int. Cl.5)**

C 07 C 45/00
C 08 G 2/00
C 07 C 47/00

Der vorliegende Recherchenbericht wurde für alle Patentansprüche erstellt

| Recherchenort | Abschlußdatum der Recherche | Prüfer |
|---|---|---|
| BERLIN | 15-01-1990 | PROBERT C.L. |